# EUROPEAN PATENT SPECIFICATION

(11) **EP 1 379 297 B1**
(45) Date of publication and mention of the grant of the patent: **04.10.2006**
(21) Application number: 02727050.3
(22) Date of filing: 05.04.2002
(51) Int. Cl.: A61M 5/178, G21F 5/018

(54) **PROCESS AND DEVICE FOR PREPARING RADIOPHARMACEUTICAL PRODUCTS FOR INJECTION**
VERFAHREN UND VORRICHTUNG ZUR VORBEREITUNG VON RADIOPHARMAZEUTISCHEN PRODUKTEN FÜR INJEKTION
PROCEDE ET DISPOSITIF POUR PREPARER DES PRODUITS RADIOPHARMACEUTIQUES A INJECTER

(30) Priority: 13.04.2001 US 283619 P
(43) Date of publication of application: 14.01.2004
(73) Proprietor: TRASIS S.A., 4000 Liège (BE)
(72) Inventor: MORELLE, Jean-Luc, B-4000 Liège (BE); PHILIPPART, Gauthier, B-4650 Grand-Rechain (BE); FREYCHET, Christèle, B-4600 Vise (BE)
(74) Representative: pronovem
(86) International application number: PCT/BE2002/000050
(87) International publication number: WO 2002/083210

(56) References cited:
- EP-A- 0 661 066
- US-A- 5 918 443
- PATENT ABSTRACTS OF JAPAN vol. 014, no. 294 (C-0732), 26 June 1990 (1990-06-26) & JP 02 095380 A (DAIICHI RAJIO ISOTOPE KENKYUSHO:KK), 6 April 1990 (1990-04-06)

## Description

### Field of the invention

The present invention is related to practices in nuclear medicine, and in particular to a process for preparing radiopharmaceutical products for injection.

### State of the art

The development of nuclear medicine, in particular in the field of diagnosis by positron emission tomography (PET), makes it necessary to review the usual methods for producing, packaging and handling radiopharmaceutical substances intended for administration to patients.

Radiopharmaceutical substances are chemical compounds labeled with radioactive isotopes, intended for medical use. Problems of existing methods and equipment are mainly due to the increase in the use of isotopes whose radiation energy is relatively high, and by the fact that a higher level of automation is required due to the short half-life of said isotopes.

Dose fractionation systems are available on the market. They make it possible to dilute a base radiopharmaceutical product, to prepare the dilute solution in vials and to place these vials in radiation shielding for transportation. The vials are then delivered to the nuclear medicine departments of hospitals. Such devices are mainly used by radiopharmaceutical production companies.

Vial dispensing systems allow to provide radio-pharmaceutical products in vials according to the common practice in most European hospitals: the vials may contain doses for several examinations or for several patients. The injection is prepared by the hospital staff: the doctor or a member of his specialist staff fills a syringe for each patient or for each examination from a "multi-dose" vial, that is to say a vial that may contain enough substance for several examinations or for several patients. This handling exposes the hospital staff on a repeated basis, giving rise to an appreciable cumulative exposure. This dose is generally limited by performing the operation behind lead protection such as a radiation-shielded glove box. The syringe may be fitted into a syringe radiation shielding.

The practice is different in the United States: hospitals receive the syringes pre-filled with the individual "patient-dose". The filling of these syringes is carried out in "radiopharmacies" (this concept is not widespread in Europe). This operation remains essentially manual: it is usually performed with handling tongs in radiation-shielded cells. It is slow and labor-intensive. The syringes are then placed, with their needle and cap, in a radiation-shielded transportation container.

In the hospital, just before injection, the syringe is removed from its transportation radiation shielding container and generally fitted into a syringe radiation shielding. This operation represents a certain level of exposure of the staff to the radiation.

It should be noted that automation of the filling of syringes is possible and that such devices exist, but they do not solve all the production problems: the fixing of the needle or a stopper is not automated, nor is the placing of the syringe in its transportation container. For these reasons, they are not widely used.

It should be noted that if the needle for the injection is already in place, as is generally the case, the syringe is not closed during transportation. The integrity of the product is not ensured during the transportation.

Document US-A-5 918 443 describes fluid leakproof package for a single-dose radiopharmaceutical-filled medical syringe. Said package is made of an inner container enclosing the complete syringe and which is in turn enclosed within a radiation-shielding outer container useful for shipment to a medical treatment location. However the configuration of this device implies that, before transportation, the syringe has to be filled and the needle capped manually, thus exposing the operator. Further, both above-mentioned containers have to be capped and uncapped respectively. Only the outer container and its cap comprise radiation-shielding material. Thus the medical staff carrying out on-site injection is further exposed during uncapping and during injection.

Alternatively, if the syringe is closed by means of a stopper, this stopper will have to be removed by the medical staff and replaced by the needle for the injection. This manipulation represents an exposure to radiation and presents a risk of radioactive contamination of the staff, and also a risk of biological contamination of the substance.

Document JP-A-02 095380 proposes a device to safely and surely transport and store a radioactive solution. This device provides improvements intended to reduce medical staff and operator exposure. For instance, for the needs of storage and transportation, only a sealed syringe body is inserted into an inner first radiation-shielding container itself enclosed in an outer second radiation-shielding container, the latter being provided with a radiation-shielding lid. The materials used for the inner container are for example lead, tungsten alloy as well as lead glass in the case where a see-through window is provided. The outer container comprises lead.

The syringe body is maintained in the device by means of a flange and a collar which are attached to the rear of the syringe body inserted in the inner shielding container.

The advantage of this solution over the previous one is that globally the shielding device is much more compact and thus less heavy. At the injection location, a plunger rod, also made of tungsten alloy to prevent radiation leakage to the rear, is screwed into a sealing gasket located inside the syringe body. Thus the syringe inserted in the inner shielding cylinder can be easily retrieved from the second shielding container.

A first drawback of this device is the need to manually remove a sealing rubber stopper at the front face of the syringe in order to place the needle, which leads to personal exposure and possible sterility loss or biological contamination. Moreover the cantilevered gasket-plunger connection is weakened owing to the weight of shielded parts.

A multi-dose radiopharmaceutical vial dispensing device is of little interest to the American market since it does not allow the automatic filling of individual patient-doses which may be used directly in hospitals according to the common practice. In the long-term, the practice of the individual patient-dose might spread beyond the United States on account of the ease offered to the hospital departments concerned.

### Problem-solution approach

The problems displayed in prior art and that need to be solved can be summarized as follows:
- the filling of a syringe as is currently performed in American, radiopharmacies, in particular for PET applications (s11 keV mean energy), is essentially manual, slow and relatively inefficient. Possible contamination or exposure of the radiopharmacies staff is thus a risk inherent to manual handling of syringes. Regarding this, automated filling is required. However usual syringes constitute a form of packaging which does not lend itself readily to automation. The design of a system for the automated filling of syringes, which would directly satisfy the need and habits of the American market, would be complex ;
- additionally, the use of individual syringes does not entirely avoid further manipulations at the hospital since they must be transferred from their transportation radiation shielding container to the syringe radiation shielding facilities ;
- pre-filled complete individual syringes, possibly comprising a needle, syringe body and plunger, are very long and require large, and thus heavy, transportation radiation shielding containers ;
- when the syringes are delivered with a set needle, the syringe body thus provided has been opened and the sterility of the product cannot be ensured anymore during transportation ;
- alternatively, when the syringes are delivered with a stopper, an additional manipulation is necessary, which exposes both the product and the operator.

### Aim of the invention

The aim of the present invention is to offer a method and a device for producing, packaging and handling injectable substances of radiopharmaceutical compounds, preferably in the form of individual patient-doses of radiopharmaceutical compounds, allowing the exposure of the staff, the number of manipulations and the need for accessory equipment to be reduced to the minimum, while maintaining isolation and sterilization of said compounds from the external environment.

### Summary of the invention

The present invention relates to a method for preparing, packaging and handling an individual patient-dose of a radiopharmaceutical compound, in particular for use as a short-lived compound in PET applications, comprising the following steps:
- filling a cartridge with said dose of radiopharmaceutical compound via a first end, the second end being closed by means of a component serving as a piston ;
- closing said cartridge at said first end by means of a closure device ;
- placing said cartridge in a radiation shielding device, comprising an inner part and an outer part, said inner part serving as radiation shielding for an operator and said outer part serving as a transportation container ;
- closing said container by means of an appropriate shielding lid ;
- transporting said container up to the place at which an injection of said radiopharmaceutical compound will take place ;
- removing the shielding lid of the container ;
- fixing a plunger to the cartridge radiation shielding ;
- extracting the cartridge and the inner part of the radiation shielding device from the outer part serving as a container, and
- placing injection means on the cartridge end which has the setting closure device.

The cartridge may be a standard cartridge, such as those used for the packaging of insulin.

Further, according to the invention, a radiation shielding device is provided, comprising an inner cylinder-like part being able to comprise said cartridge, and an outer cylinder-like part, serving as an additional shielding for transportation. The inner part is capable to be placed inside the outer part. The outer part is able to be closed by a shielding lid. The inner cylinder-like part is preferably made of high density metal such as tungsten or a tungsten-based alloy. The outer cylinder-like part and the shielding lid are preferably made of a high density metal such as lead, tungsten, a high lead content alloy or a high tungsten content alloy.

Also, according to the invention, a plunger to be used in the method is provided, said plunger comprising a sliding rod, a push button, means for fixing said plunger to the inner part of the radiation shielding device and support wings for the fingers.

Also according to the invention, an injection means to be used in the method is provided, said injection means comprising a needle or a luer fitting connected to a spike, said spike being capable to pierce the set closure of the cartridge in a sterile manner and to enter in contact with said radiopharmaceutical compound, when said cartridge is inside the cartridge radiation shielding. The spike is protected in a sterile manner by an elastomeric sleeve until set closure of cartridge has been pierced.

### Brief description of the drawings

FIGS. 1a and 1b represent an open cartridge with its setting stopper and a closed cartridge respectively.

FIG. 2 represents, in a cutaway view, a radiation-shielding device

FIGS. 3a, 3b and 3c represent a plunger, viewed separately, viewed assembled on the cartridge radiation shielding and viewed during extraction from the shielding device used for the transportation respectively.

FIGS. 4a, 4b and 4c represent a first embodiment of injection means comprising a spike and a needle, with and without its sleeve and caps respectively.

FIGS. 5a, 5b and 5c represent a second embodiment of injection means comprising a spike and a male luer-lock device, with and without its cap and plug respectively.

FIG. 6 is a view of the ready-to-use injection device.

### Detailed description of several preferred embodiments of the present invention

The present invention is related to a method and devices for preparing, packaging and handling individual doses of an injectable radiopharmaceutical compound. The method combines and incorporates the functions of filling (production) and transportation (packaging) and use while at the same time allowing the production to be easily automated.

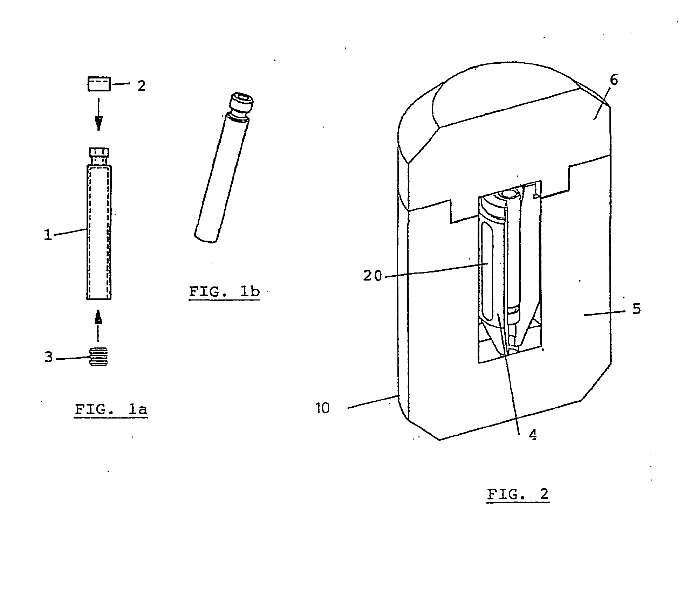

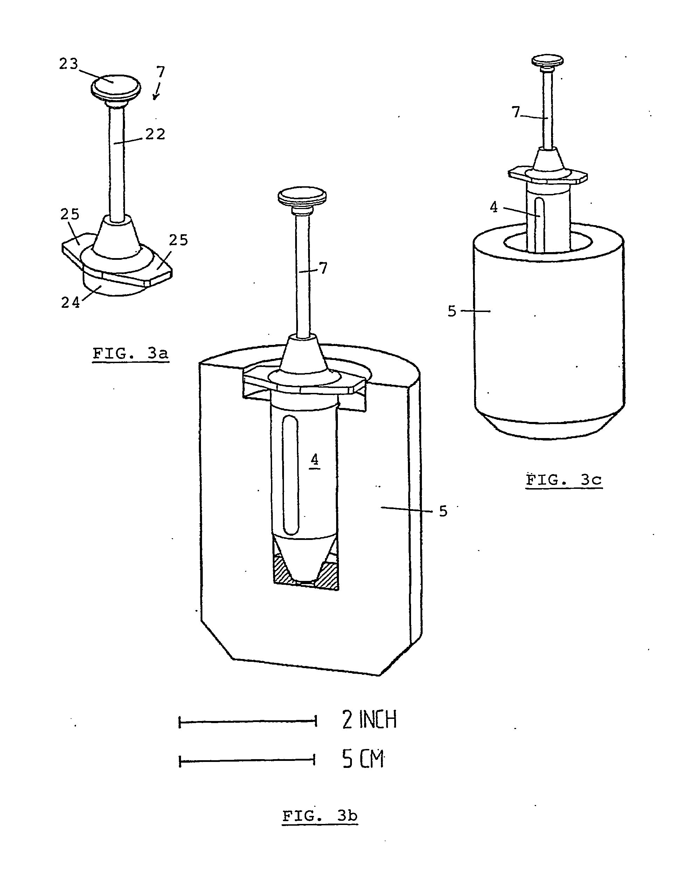

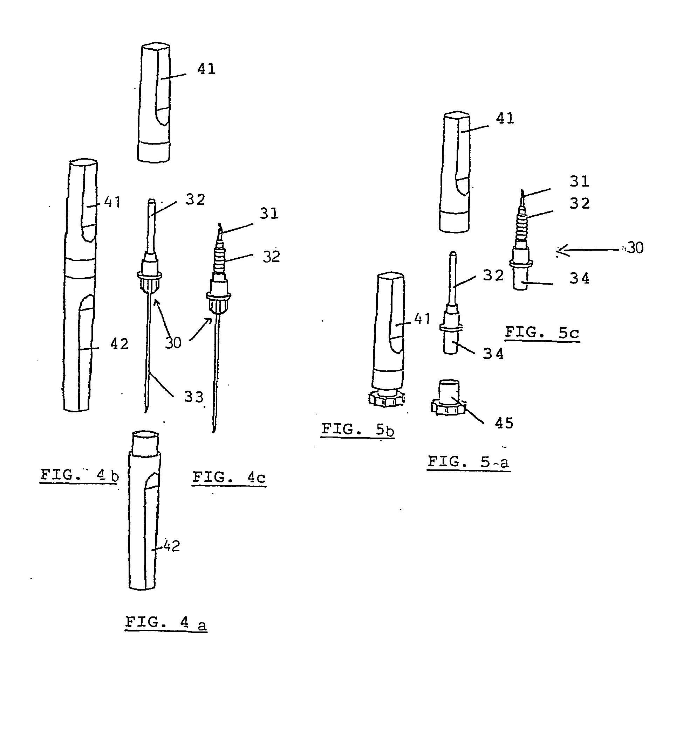

The method of the invention comprises the following steps:
- filling a "cartridge" 1 with an injectable substance of a radiopharmaceutical compound (FIG. 1) ;
- closing said cartridge 1 at one end by means of a setting elastomeric (rubber) stopper 2 to be capable of being pierced, such as a septum. The cartridge comprises a rubber piston 3 at the other end. The filling and closing may be performed automatically using a suitable dispensing device. A cartridge as is known to contain insulin may be used for this purpose ;
- placing the cartridge in a radiation shielding device 10 consisting of two concentric parts (FIG. 2): a removable inner part 4, called a "cartridge radiation shielding", consisting of a tube made of dense material which will also serve as protection for the medical staff during the injection (thus fulfilling the function of syringe radiation shielding), and an outer part 5 consisting of a "radiation-shielding container" whose size and thickness are suitable for transportation according to the nature of the substance contained in the cartridge. The cartridge and its shielding can be easily placed in this radiation shielding container by means of an automated device ;
- fitting a shielding lid 6 on said container 5 ;
- transporting said container 5 to the place where the substance will be administered ;
- removing the lid 6 from the transportation container 5 and attaching by clipping or screwing a plunger 7 to the cartridge radiation shielding 4 (FIGS. 3) ;
- extracting the cartridge radiation shielding 4 from the transportation container 5 by means of this plunger 7. At this stage, the user has in his hands the equivalent of a filled radiation-shielded syringe which lacks only the needle ;
- placing a spike 31 by piercing the elastomeric stopper 2 opposite to the piston 3. The spike may be a double needle of VACUTAINER® type (from Becton Dickinson) as used for taking blood (FIGS. 4) or the spike may be connected to a male luer fitting (luer-lock or -slipper) adapted for any type of subsequent connection of the device. This spike, which is protected from any dust by an elastomeric (rubber) sleeve 32, is installed by piercing the septum 2 of the cartridge 1 ;
- removing the cap 42 from the intravenous (iv) needle or the plug 45 of the luer fitting in order to carry out the injection (FIGS. 4 and 5).

The main components used in the method of the invention are described hereunder. Some of them are existing parts, used here for the purpose of the invention. Others are specific to the invention.

The cartridge (FIG. 1) is a type of tube 1 closed at one end by means of a rubber piston 3 which can slide inside the tube, and fitted at the other end with a system of closure by setting a rubber stopper (not shown) used as a septum 2 with a crimped aluminium ring.

The cartridge can be any standard commercially available single-use component such as for example "1.5 ml Cartridge, flint type I glass" from Forma Vitrum A.G. (Switzerland) or "1.8 ml Cartridge, ref. No. 112" from Nuova Ompi (Italy).

The cartridge radiation shielding 4 (FIG. 2) is a cylindrical component made of a dense radiation shielding alloy, for example a tungsten-based alloy. It is hollow, with its inside diameter adapted to the cartridge. The thickness of the wall is adapted to the weight and radiation shielding constraints. The cartridge radiation shielding is reusable and may be placed in the transportation radiation shielding container 5. A window 20 may be provided in the shielding 4 to allow the cartridge to be seen through the lateral face. A conical inlet may be provided to facilitate the automatic insertion of the cartridge 1. The cartridge radiation shielding 4 is a purpose-designed and purpose-made component, specifically for this invention.

The transportation container 5 is a cylinder made of dense material, preferably lead, intended to protect the environment from the radiation emitted by the contents of the cartridge during the transportation and storage. The container is fitted with a shielding lid 6. The internal dimensions are adapted to those of the cartridge radiation shielding 4. The thickness of the walls is adapted to the nature and intensity of the radioactive source.

The plunger 7 (FIGS. 3) serves to push the cartridge piston 3 during the injection and to extract the cartridge radiation shielding 4 from the transportation container 5. It is composed of a sliding rod 22 fitted at one end with a push button 23 for the thumb and at the other end with a back face to enter into contact with the cartridge piston 3. The fixed part comprises a system for rapid attachment 24, for example by screwing or clipping, to the cartridge container described above and is fitted with two support wings 25 for the fingers. This component is reusable. It is a purpose-designed and purpose-made assembly, specifically intended for this use.

According to a first preferred embodiment, as shown in FIGS. 4a, 4b and 4c, injection means 30 essentially consist in a device presenting a needle 33, such as an iv needle, connected to a spike 31. The spike is designed to be able to pierce the set closure 2 of the cartridge 1 when said cartridge is still inside the cartridge radiation shielding 4.

Advantageously, said spike is protected by an elastomeric sleeve 32 and both spike 31 and needle 33 are covered before use by adequate protective caps 41 and 42.

According to another preferred embodiment, as shown in FIGS. 5a, 5b and 5c, the injection means 30 essentially consist in a device comprising a spike 31 as described in the previous embodiment and a luer-lock fitting 34 able to be closed by a plug 45.

Again, the spike 31 may advantageously be protected by an elastomeric sleeve 32. The spike may also be covered before use by a protective cap 41.

The injection means 30 is a device permitting rapid fixing of a needle or luer lock using a screw pitch (threaded part) or a clip (not shown). The fixing is made to the lower end of the radiation-shielding 4. The spike 31 is designed to be able to pierce the set closure 2 of the cartridge when the latter is inside the cartridge radiation shielding 4. The opposite end of the injection means is either an iv needle 33 (FIGS. 4) or a male luer-lock fitting 34 (FIGS. 5). The direct use of a standard single-use spike/needle of VACUTAINER® type as used for taking blood is the most practical (Becton Dickinson, Vacutainer needle, ref. 36-0213).

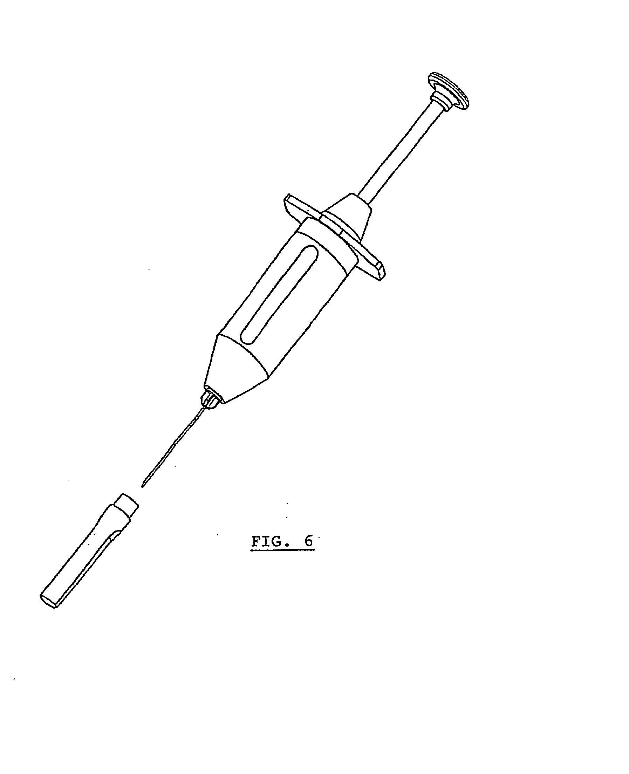

In FIG. 6 is described the manner in which the cartridge radiation shielding 4 provided with its plunger 7 and internally with a cartridge 1 is fitted with the injection means such as the spike/needle 30 device described in FIG. 4a. Once the set closure 2 has been pierced by the spike 31, through a lower opening in said shielding 4, the protective cap 42 only needs to be removed in order to proceed with the injection.

## Claims

1. Device to be used in the automated preparation and packaging and in the further handling of a radiopharmaceutical compound individual dose, comprising the following set of elements
- a cartridge intended to contain said individual dose, provided at a first end with a closing element (2) and at a second end by means of a component serving as a piston (3) ;
- a radiation-shielding container (10) comprising an inner cylinder-like part (4) capable to enclose said cartridge (1), and an outer cylinder-like part (5), serving as an additional shielding for transportation, said outer part being capable to enclose said inner part, said outer part being provided with a shielding lid (6) ;
- a plunger (7) to be fitted to the cartridge (1) at the time of an injection, while said cartridge is still inside said inner cylinder-like part (4) of the radiation shielding container (10), provided with a sliding rod (22) capable to contact said piston (3) of the cartridge (1), with a push button (23) and with support wings (25) for the fingers ;
- an injection means (30) to be fitted to the first end of the cartridge (1) at the time of an injection, while said cartridge is still inside said inner cylinder-like part (4) of the radiation shielding container (4), **characterised in that**
- the plunger is provided with fixing means (24) to said inner cylinder-like part (4) of the radiation shielding container (10) ;
- the closing element (2) is a septum, preferably an elastomeric stopper and the injection means (30) is capable to pierce the closing element (2) of the cartridge (1) in a sterile manner and to enter in contact with said radiopharmaceutical compound.

2. Device according to Claim 1, wherein the fixing means (24) of the plunger comprise a rapid attachment device such as screwing or clipping means.

3. Device according to Claim 1, wherein said injection means (30) comprises inwardly a spike (31) and outwardly a needle (33) or a luer fitting (34) in connection to the spike (31), preferably of the VACUTAINER® type.

4. Device according to Claim 1, wherein said spike (31) is protected in a sterile manner by an elastomeric sleeve (32) until said closing element (2) of cartridge (1) has been pierced.

5. Device according to Claim 3 or 4, wherein the injection means elements (30) are covered by a protective cap (41,42) before use.

6. Device according to Claim 1, wherein the injection means (30) are designed for rapid attachment to the cartridge such as screwing or clipping.

7. Device according to Claim 1, wherein said cartridge is a standard cartridge of the type used for the packaging of insulin.

8. Device according to Claim 1, wherein it is suitable for using a short-lived Positron Emission Tomography (PET) compound.

9. Device according to Claim 1, wherein said inner cylinder-like part (4) is made of high density metal such as tungsten or a tungsten-based alloy.

10. Device according to Claim 1, wherein said outer cylinder-like part (5) and said shielding lid (6) are made of a high density metal such as lead, tungsten, a high lead content alloy or a high tungsten content alloy.

11. Device according to Claim 1, wherein a see-through window (20) is provided in the shielding (4).

12. Use of the device, according to anyone of Claim 1 to 11, for automatically preparing and packaging and for handling an individual dose of a radiopharmaceutical compound, comprising the following steps:
- filling the cartridge (1) with said dose of radiopharmaceutical compound via its first end, its second end remaining closed by means of the piston (3) ;
- closing said cartridge (1) at said first end by means of the closure device (2) ;
- placing said cartridge (1) in the radiation shielding device (10), comprising an inner part (4) and an outer part (5), said inner part serving as radiation shielding for an operator and said outer part serving as a transportation shielding container ;
- closing said shielding container (5) by means of the shielding lid (6) ;
- transporting said container up to the place at which an injection of said radiopharmaceutical compound will be carried out ;
- removing the shielding lid (6) of the container ;
- fixing the plunger (7) to the inner part (4) of the shielding device (10) ;
- extracting the inner part (4) of the radiation shielding device (10), enclosing the cartridge, from the outer part (5), and
- placing injection means (30) on the cartridge end which is provided with the closing element (2), said cartridge (1) being still enclosed in the inner part (4) of the shielding device (10).

## Patentansprüche

1. Ein für die automatisierte Vorbereitung und Verpackung sowie zur weiteren Handhabung von radiopharmakologischen Einzel-Präparatdosen zu verwendendes Gerät, das die folgenden Bauteile enthält:
- eine Patrone, die die besagte Einzeldosis aufnehmen soll und an einem ersten Ende mit einem Verschlusselement (2) ausgestattet ist und ferner an einem zweiten Ende über ein Bauteil verfügt, das als Kolben (3) dient;
- einen Strahlenschutzbehälter (10), der einen inneren zylinderartigen Teil (4) enthält, der die besagte Patrone (1) umschließen kann, sowie einen äußeren zylinderartigen Teil (5), der als zusätzliche Transportabschirmung dient; der besagte äußere Teil kann den besagten inneren Teil umschließen und ist mit einem abschirmenden Deckel (6) ausgestattet;
- einen einfach wirkenden, an der Patrone (1) zum Zeitpunkt der Injektion zu befestigenden Druckstempel (7), während sich die besagte Patrone zu diesem Zeitpunkt immer noch im inneren zylinderartigen Teil (4) des Strahlenschutzbehälters (10) befindet; der Druckstempel ist mit einem Schiebergestänge (22) ausgestattet, das besagten Kolben (3) der Patrone (1) berühren kann, ferner mit einem Drucktaster (23) und mit Stützflügeln (25) für die einfachere Handhabung mit den Fingern;
- einer Injektionsvorrichtung (30), die am ersten Ende der Patrone (1) zum Zeitpunkt der Injektion angebracht werden kann, während sich die besagte Patrone zu diesem Zeitpunkt immer noch im inneren zylinderartigen Teil (4) des Strahlenschutzbehälters (10) befindet, sowie **dadurch gekennzeichnet, dass** der Druckstempel mit einer Befestigungsvorrichtung (24) am inneren zylinderartigen Teil (4) des Strahlenschutzbehälters (10) ausgestattet ist;
- Beim Verschlusselement (2) handelt es sich um ein Trennelement, vorzugsweise um einen Elastomerstopfen; die Einspritzvorrichtung (30) kann das Verschlusselement (2) der Patrone (1} in einer sterilen Weise durchstechen und zum besagten radiopharmakologischen Präparat vordringen.

2. Ein Gerät gemäß Anspruch 1, bei dem die Befestigungsvorrichtung (24) des Druckstempels eine Schnellbefestigung wie z.B. eine Schraub- oder Klippvorrichtung umfasst.

3. Ein Gerät gemäß Anspruch 1, bei dem die besagte Injektionsvorrichtung (30) im Inneren einen Dorn (31) und außen eine Nadel (33) bzw. ein Luer-Fitting (34) in Verbindung mit dem Dorn (31), vorzugsweise vom Typ VACUTAINER® enthält.

4. Ein Gerät gemäß Anspruch 1, bei dem der besagte Dorn (31) in steriler Weise durch eine elastomere Hülse (32) geschützt ist, bis das besagte Verschlusselement (2) der Patrone (1) durchstochen wurde.

5. Ein Gerät gemäß Anspruch 3 oder 4, bei dem die Bauteile der besagten Injektionsvorrichtung (30) vor der Verwendung von einer Schutzkappe (41, 42) abgedeckt werden.

6. Ein Gerät gemäß Anspruch 1, bei dem die Injektionsvorrichtung (30) für eine schnelle Befestigung an der Patrone wie z.B. durch eine Schraub- oder Klippvorrichtung ausgelegt ist.

7. Ein Gerät gemäß Anspruch 1, bei dem es sich bei der Patrone um eine Standardpatrone in der Art handelt, wie sie für Insulinverpackungen verwendet werden.

8. Ein Gerät gemäß Anspruch 1, das für die Verwendung eines kurzlebigen Präparats für die Positronen-Emissions-Ttomographie (PET) geeignet ist.

9. Ein Gerät gemäß Anspruch 1, bei dem der besagte innere zylinderartige Teil (4) aus einem hochdichtem Material wie z.B. Wolfram oder einer Wolframlegierung gefertigt ist.

10. Ein Gerät gemäß Anspruch 1, bei dem der besagte äußere zylinderartige Teil (5) sowie der besagte abschirmende Deckel (6) aus einem hochdichtem Material wie z.B. Blei, Wolfram, einer stark bleihaltigen Legierung oder einer Legierung mit hohem Wolframgehalt gefertigt ist.

11. Ein Gerät gemäß Anspruch 1, bei dem die Abschirmung (4) über ein Schauglass (20) verfügt.

12. Die Verwendung des Geräts gemäß einem der Ansprüche 1 bis 11 für die automatische Vorbereitung und Verpackung sowie für die Handhabung von radiopharmakologischen Einzel-Präparatdosen, die die folgenden Schritte enthält:
- Das Auffüllen der Patrone (1) mit der besagten Dosis eines radiopharmakologischen Präparats über sein erstes Ende, während das zweite Ende durch den Kolben (3) verschlossen bleibt;
- Das Verschließen der besagten Patrone(1) am besagten ersten Ende mithilfe des Verschlusselements (2);
- Das Einsetzen der besagten Patrone (1) in den Strahlenschutzbehälter (10), der einen inneren (4) und einen äußeren Teil (5) umfasst; besagter innerer Teil dient als Strahlungsschutz für den Verwender, während besagter äußerer Teil als Strahlenschutzcontainer für den Transport dient;
- Das Verschließen des besagten Strahlenschutzbehälters (5) mithilfe des abschirmenden Deckels (6);
- Der Transport des besagten Behälters bis zu dem Ort, an dem die Injektion des besagten radiopharmakologischen Präparats durchgeführt wird, indem der abschirmende Deckel (6) des Behälters entfernt wird;
- Das Befestigen des Druckstempels (7) am inneren Teil (4) der abschirmenden Vorrichtung (10);
- Das Herausziehen des inneren Teils (4) der Strahlenschutzvorrichtung (10), die die Patrone umschließt, aus dem äußeren Teil (5), sowie das Anbringen der Injektionsvorrichtung (30) an dem mit dem Verschlusselement (2)ausgestattetem Patronenende; hierbei ist die besagte Patrone (1) immer noch vom inneren Teil (4) der Strahlenschutzvorrichtung (10) umschlossen.

## Revendications

1. Dispositif destiné à être utilisé pour la préparation et l'emballage automatisés et dans la manipulation ultérieure d'une dose individuelle d'un composé radiopharmaceutique comprenant l'ensemble d'éléments suivant :
- une carpule destinée à contenir ladite dose individuelle, munie à une première extrémité d'un élément de fermeture (2) et à une seconde extrémité par un composant servant de piston (3) ;
- un conteneur anti-radiation (10) comprenant une partie cylindrique interne (4) capable d'entourer ladite carpule (1) et une partie cylindrique externe (5) servant de protection anti-radiation supplémentaire pour le transport, ladite partie externe étant capable d'entourer ladite partie interne, ladite partie externe étant munie d'un couvercle anti-radiation (6) ;
- un piston (7) destiné à être inséré dans la carpule (1) au moment de l'injection, tandis que ladite carpule est toujours à l'intérieur de ladite partie cylindrique interne (4) du conteneur anti-radiation (10), muni d'une tige coulissante (22) capable d'entrer en contact avec ledit piston (3) de la carpule (1) avec un bouton-poussoir (23) et des ailes de support (25) pour les doigts ;
- un moyen d'injection (30) destiné à être inséré à la première extrémité de la carpule (1) au moment de l'injection, tandis que ladite carpule est toujours à l'intérieur de ladite partie cylindrique interne (4) du conteneur anti-radiation (4),
**caractérisé en ce que**
- le piston est muni de moyens de fixation (24) à ladite partie cylindrique interne (4) du conteneur anti-radiation (10) ;
- l'élément de fermeture (2) est un septum, de préférence un bouchon élastomérique et le moyen d'injection (30) est capable de percer l'élément de fermeture (2) de la carpule (1) de manière stérile et d'entrer en contact avec ledit composé radiopharmaceutique.

2. Dispositif selon la revendication 1, **caractérisé en ce que** le moyen de fixation (24) du piston comprend un élément de fixation rapide tel que des moyens de vissage ou de clipsage.

3. Dispositif selon la revendication 1, **caractérisé en ce que** ledit moyen d'injection comprend à l'intérieur un spike (31) et à l'extérieur une aiguille (33) ou un embout Luer-Lock (34) connectés au spike (31), de préférence du type vacutainer® .

4. Dispositif selon la revendication 1, **caractérisé en ce que** ledit spike (31) est protégé de manière stérile par un manchon élastomérique (32) jusqu'à ce que l'élément de fermeture (2) de la carpule ait été percé.

5. Dispositif selon la revendication 3 ou 4, **caractérisé en ce que** les éléments des moyens d'injection (30) sont couverts par un capuchon de protection (41,42) avant usage.

6. Dispositif selon la revendication 1, **caractérisé en ce que** les moyens d'injection (30) sont conçus pour la fixation rapide à la carpule tels que vissage ou clipsage.

7. Dispositif selon la revendication 1, **caractérisé en ce que** ladite carpule est une carpule standard du type utilisé pour le conditionnement de l'insuline.

8. Dispositif selon la revendication 1, **caractérisé en ce qu'**il est adapté à l'utilisation d'un composé à courte durée de tomographie à émission de positron (PET).

9. Dispositif selon la revendication 1, **caractérisé en ce que** ladite partie cylindrique intérieure (4) est faite d'un métal à haute densité tel que le tungstène ou un alliage à base de tungstène.

10. Dispositif selon la revendication 1, **caractérisé en ce que** ladite partie cylindrique externe (5) et ledit couvercle anti-radiation (6) sont faits d'un métal à haute densité tel que le plomb, le tungstène, un alliage à haute teneur en plomb ou un alliage à haute teneur en tungstène.

11. Dispositif selon la revendication 1, **caractérisé en ce qu'**une fenêtre transparente (20) est prévue dans la protection anti-radiation (4).

12. Utilisation d'un dispositif selon l'une quelconque des revendications 1 à 11, pour la préparation et le conditionnement automatiques et pour la manipulation d'une dose individuelle d'un composé radiopharmaçeutique comprenant les étapes suivantes :
- remplissage de la carpule (1) avec ladite dose du composé radiopharmaceutique par sa première extrémité, sa seconde extrémité restant fermée par le piston (3),
- fermeture de ladite carpule (1) à ladite première extrémité par un dispositif de fermeture (2),
- positionnement de ladite carpule (1) dans un dispositif anti-radiation (10), comprenant une partie interne (4) et une partie externe (5), ladite partie interne servant de protection anti-radiation pour l'opérateur et ladite partie externe servant de conteneur anti-radiation de transport,
- fermeture dudit conteneur anti-radiation (5) par le couvercle anti-radiation (6),
- transport dudit conteneur jusqu'à l'endroit où l'injection dudit composé radiopharmaceutique aura lieu,
- extraction du couvercle anti-radiation (6) du conteneur,
- fixation du piston (7) à la partie interne (4) du dispositif anti-radiation (10),
- extraction de la partie interne (4) du dispositif anti-radiation (10) entourant la carpule de la partie externe (5), et
- positionnement des moyens d'injection (30) sur l'extrémité de la carpule qui présentant l'élément de fermeture (2), ladite carpule (1) étant toujours entourée par la partie interne (4) du dispositif anti-radiation (10).
